# EUROPEAN PATENT SPECIFICATION

(11) **EP 0 456 885 B1**
(45) Date of publication and mention of the grant of the patent: **15.02.1995**
(21) Application number: 90119775.6
(22) Date of filing: 15.10.1990
(51) Int. Cl.: A61F 13/15, A61F 5/44

(54) **Child's training pants and process of manufacture**
Erziehungshöschen für Kinder und Herstellungsverfahren
Couche-culotte pour enfant et procédé de fabrication

(30) Priority: 16.05.1990 US 522841
(43) Date of publication of application: 21.11.1991
(73) Proprietor: POPE & TALBOT COMPANY, Shenandoah, Georgia 30265 (US)
(72) Inventor: Weeks, L. Jane, Newnan, Georgia 30265 (US); Calderon, Maria M., Sharpsburg, Georgia 30277 (US); Minetola, James A., Peachtree City, Georgia 30269 (US)
(74) Representative: Finck, Dieter, Dr.Ing.

(56) References cited:
- EP-A- 0 119 827
- EP-A- 0 320 991
- EP-A- 0 357 298
- US-A- 4 795 510
- US-A- 4 826 499

## Description

The invention relates to a process for providing a disposable training pant for adolescent children and incontinent adults comprising the steps of providing a web of backing material, moving said web along an assembly machine and applying discrete sections of adhesive on said web as well as a disposable training pant provided by said process.

The disposable baby diaper business is of tremendous proportions, accounting for annual sales of more than 16 billion units for a value in excess of $4,000,000,000.

Such products are usually the one-piece shaped elastic leg system popular in the United States or, in many cases, the two-piece (Swedish) system preferred by many in Europe.

Such products are primarily for use on infants from birth to two years of age, by which time most of the children are "toilet trained" and no longer need a disposable diaper.

However, it has been recently recognized that a substantial number of children who have "grown out of diapers" and are assumed to be "toilet trained" have not reached that stage of development, and a substantial demand exists for an intermediate garment which has popularly been called the "training pant".

It is important from a psychological standpoint that this product be different from "baby diapers", because the child who is being toilet trained no longer believes he or she is a baby and has grown up to become a "big kid".

Thus it is desirable that the product appear to be more like the adults' underwear and, even more preferably, of the "jockey" or shorts-type rather than the open, loose-leg or "Bermuda shorts" type.

In any event, the product should be cosmetically acceptable and constructed in such a way that it is not bulky and yet fits properly and is easy to put on and take off.

In connection with the manufacturing of disposable absorbent articles, such as diapers, training pants, incontinence garments and the like the US-A-4,795,510 discloses a method for adhering individual patches of substrate material to spaced-apart regions of a moving web layer, comprising the steps of: moving a selected web layer; providing a selected substrate material; depositing a selected coating of hot-melt adhesive onto said substrate material; automatically maintaining a selected cross-directional registry between said substrate material and said deposited coating of adhesive; selectively cooling said coated substrate material; engaging said coated substrate material to feed it at a selected rate to a segmenting step; segmenting said coated substrate material into individual patches; spatially segregating said patches by a selected amount; adhesively securing said segregated patches onto selected spaced-apart regions of said moving web layer; and selectively retracting said coated substrate material by a selected amount to draw said coat substrate material away from a striking point of said segmenting step when said feeding of the coated substrate material is disengaged and stopped, thereby substantially preventing undesired segmenting of said coated substrate material.

This method is considered as being generic.

EP-A-0 320 991 refers to a disposable incontinence garment or training pant for absorbing human discharge. This article includes an absorbent assembly comprising a liquid-impervious outer cover, a liquid-pervious liner and an absorbent medium therebetween; said absorbent assembly further comprising generally opposite side edges and generally opposite end edges, a pair of stretchable side panels being joined to each one of said side edges to form with said absorbent assembly a waist opening and a pair of leg openings and a gathering means being joined along at least a portion of a respective one of each said leg openings for gathering said portion, whereby said stretchable side panels provide generally inwardly directly force vectors against a wearer to maintain said garment snugly against the wearer's body and said absorbent assembly snugly in place against the crotch area both before and after a discharge, and said gathering means provide elasticity about said leg openings to prevent leakage thereat.

Because the prior art fails to provide the attactive cosmetic, functionally-effective results desired, it is a specific object of the present invention to provide a superior, efficient and economically-functional process for tee production of a training pant at high speeds on equipment similar to that which has been known in the art to produce baby diapers, which process will enable the product to be sold at a low price, which justifies its disposability after one wearing, as well as - provided by said process - a highly attractive training pant for children who have grown out of diapers, but are not yet toilet trained and a more effective containment device for body exudates when worn by incontinent persons, such a children who have not yet been toilet trained or adults who are subject to adult incontinence problems.

This object is obtained with the process of the generic kind by providing a web of absorbent core material, separating said core web into sections, depositing a waist elastic member on each of said sections of adhesive, applying a discreet area of barrier material to said web of backing material, depositing a section of said core material upon the aforementioned barrier material, providing a second web of pervious coverstock material, applying discreet sections of adhesive material transversely on said second web to define at least a pair of leg elastic securement areas and a pair of frontal elastic securement areas, providing a plurality of leg elastic members and a plurality of frontal elastic members, depositing said leg elastic members and said frontal elastic members upon the adhesive in the respective securement areas, inverting said second web and moving it onto the web of pervious backing material, in alignment therewith, and in synchronism therewith, so that the aforementioned leg and frontal elastic members are disposed one on each side of the section of core material, bonding said webs together, folding said bonded webs longitudinally in half and aligning the opposed edges thereof, removing a section of the bonded webs along the fold line, and cutting said webs along a line transversely of said webs at the middle of said removed section and sealing the webs on each side of said cutting line to provide individual training pants.

Preferably said elastic members are made of heat activatible plastic material which is flat and non-elastic when applied to the respective webs.

It is convenient to pass said individual garments through a heating chamber so as to activate the heat-activatable elastic.

Advantageously, the adhesive material is a hot-melt adhesive.

The bonding of the two webs can be accomplished by using a hot-melt material or by mechanically crimping the two webs together, or by an ultrasonic means, or by sewing.

Preferably the cutting and sealing of the folded webs are accomplished simultaneously.

It is convenient that the heating chamber is at a temperature of 80 and 100°C (180° and 210° F) and the residence time of the pants in the chamber is between 8 to 12 seconds.

Preferably the temperature of the heating chamber is 93°C (200° F) and the residence time of the pants in the chamber is 10 seconds.

A training pant for adolescent children and incontinent adults provided by the above-mentioned process has a front waist portion with a frontal end, a rear waist portion with a rear end, a crotch portion, side portions and leg openings in said side portions adjacent said crotch portion, a backsheet, a porous topsheet, an absorbent core having a major and a minor dimension and disposed between the topsheet and the backsheet and between the leg openings, at least one leg elastic member between each major dimension of the absorbent core and its adjacent leg opening, one end of each of said leg elastic members terminating in the front waist portion, a pair of frontal elastic tapes in the front waist portion, at least a portion of which tapes lie above the terminal ends of the leg elastic members and differs from the generic prior art according to EP-A-0 320 991 by a barrier material disposed between the core and the backsheet.

Preferably the frontal elastic tapes are separated laterally a distance greater than the distance between the leg elastic members.

It is advantageous that the tension in the frontal elastic tapes is different from the tension in the leg elastic members.

The training pant may include at least one waist elastic member in the front waist portion between the frontal elastic tapes and the frontal end of the pant.

The training pant may also include a waist elastic member in the rear waist portion above the terminal ends of the leg elastic members and the rear end of the pant. Preferably the absorbent core is rectangular. Conveniently the backsheet is a porous material. The porous backsheet and the porous topsheet may both be formed of hydro-entangled nonwoven webs. Preferably the backsheet is a cotton material. The leg elastic members should be between 30 and 35 cm (12 and 14 inches) long. The frontal elastic members should be between 10 and 18 cm (4 and 7 inches) long. Usually the leg elastic members are 25 cm (10 inches) long and the frontal elastic members are 15 cm (6 inches) long. Preferably the absorbent core is 9 cm (3.5 inches) wide and 45 cm (17.5 inches) long. It is convenient that the absorbent core is a combination of superabsorbent material, cellulose pulp and thermally-bondable fibers. Preferably the elastic members are a heat shrinkable thermal-elastic material. The backsheet and the topsheet can be made of a polyester spunlace material.

A clear hot-melt adhesive material can be used as a barrier material.

The process for producing the garment of the present invention is a relatively uncomplicated straight-line process which, in one aspect includes unwinding a breathable pervious backing sheet, onto which are applied the necessary adhesives, waist elastics, fluid barrier, and absorbent core.

A second portion of the apparatus and process includes the unwinding of a pervious nonwoven coverstock material, to which are applied the adhesives and the elastic mechanisms for the leg elastics and the frontal elastics.

In both of the unwind and accumulating operations, the lengthwise direction of the diaper is disposed cross-machine or at right angles to the flow of the material as the various elements are accumulated.

Subsequently, the coverstock with its assembled elastic members is laid down upon the backing strip with its assembled members to combine the product. Thereafter the assembled webs are folded longitudinally in half and portions thereof joined together at spaced intervals and portions removed also at spaced intervals to provide individual and discreet training pants.

The so-separated garments pass through a heating chamber where the elastic members are activated to provide the finished product and, from there, pass on to a packaging assembly process.

So in the present invention, a disposable training pant is provided which may have a breathable, cloth-like backing sheet or outcover, impervious in the fluid-contacting area, a cloth-like, pervious body-contacting topsheet or inner cover, with a highly integrated absorbent pad disposed between the two cover materials.

In addition to the absorbent matt or core, the garment also includes a leg elasticating mechanism, a waist elasticating mechanism, and a frontal elasticating mechanism to provide a comfortable, form-fitting garment, which is easily drawn over the legs and around the body by a youngster without the need for application of the garment to the body of the youngster by a parent or a care-giver. When worn, the garment gives every appearance of being very similar to universally-acceptable types of underwear, while yet providing protection against accidental discharges which occur during the "toilet training" period.

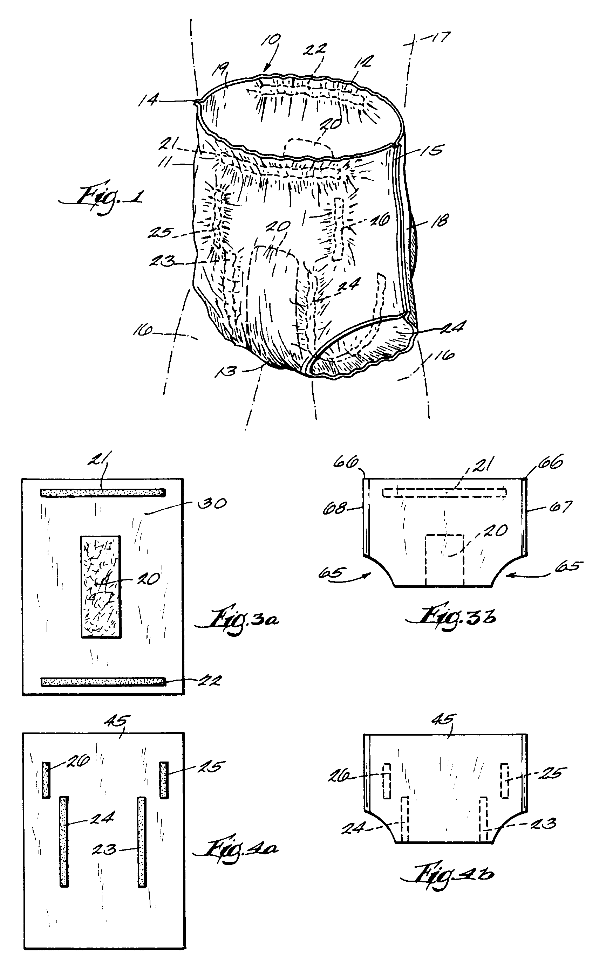

The invention is further illustrated by means of the accompanying drawings in which Figure 1 is a perspective view of the training pant of the present invention as it would appear when worn by a child,
Figure 2 is a schematic flow chart of the assembly of the various component parts of the training pant to provide the finished product,
Figures 3a and 3B illustrate the disposition of the elements on the backing sheet at various stages during the process,
Figures 4a and 4b illustrate the nonwoven coverstock with its various assembled components at separate stages of the assembly process, and
Figure 5 is a diagramatic conceptual view of one of the training pants when laid flat, prior to folding the pant in half, and prior to attaching the sides and prior to heat-shrinking the elastic members.

Referring now to Figure 1, there is shown a garment or training pant 10, as it would appear and worn by a child, disposed around the torso (i.e., the waist) and above the legs of the child. The garment includes a front portion 11 and a back portion 12 connected by a crotch portion 13. The front portion 11 and the back portion 12 are joined together along side flanges 14 and 15 respectively to provide a unitary undergarment which is drawn up upon the body over the legs 16 to the waist 17 of the infant.

The garment includes an outer cover 18 and an inner pervious body-contacting liner 19 with an absorbent pad 20 disposed between the two materials and arranged to lie in the crotch portion of the pant around the genitalia of the infant. As designed, this product is equally effective for female children as well as male children.

The garment also may include a front waist elasticating member 21 and a back waist elasticating member 22, as well as a pair of leg elasticating members 23 and 24.

Unique to the garment of the present invention is a pair of frontal elastics 25 and 26.
it is well-known in prior art to have waist elastics in disposable diapers (similar to the waist elastics 21 and 22 of the present invention) and to have leg elasticating mechanisms such as the leg elasticating mechanism in 23 and 24 of the present invention. It is previously known in the art that these elasticating mechanisms may be provided from material which is non-elastic and flat when applied to the garment, that such elements may thereafter be elasticated by subjecting them to heat. Such heat-shrinkable materials are well known.

Nevertheless, the garment of the present invention, for the first time, provides a separate pair of frontal elastic members 25 and 26 in the front waist area, a major portion of which is disposed above the terminal portions of the leg elastics 23 and 24 and provides, for the first time, a garment which is uniquely cosmetically able to fit on children of different ages and different body dimension.

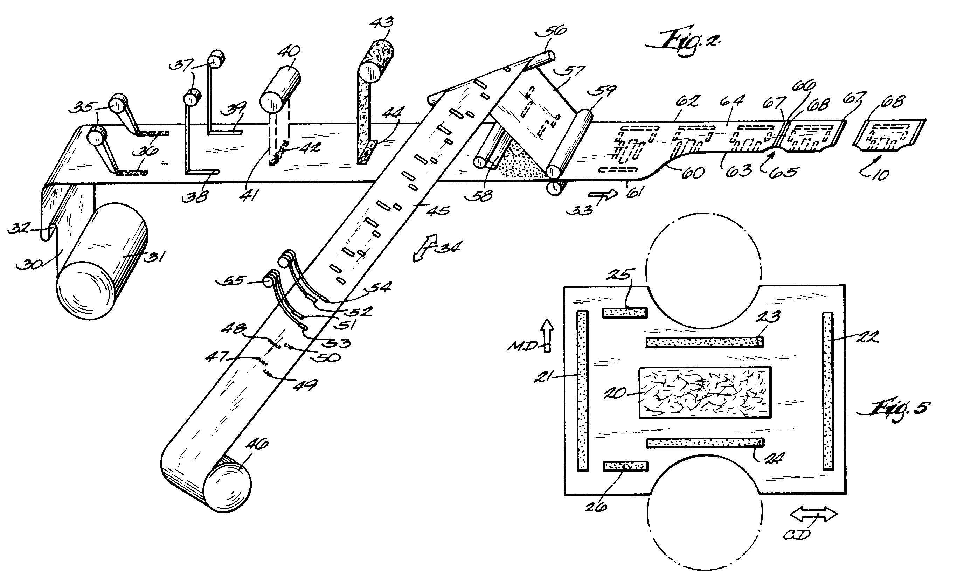

The construction of the garment of the present invention can be better understood by reference to Figure 2, which is a flow diagram depicting the assembly of the component parts of the garment.

In Figure 2, reference 30 refers to a backsheet material which may be a spun-lace or hydro-entangled web, all as is well-known in the art. The backsheet 30 unwinds from a roll 31 through a tension control mechanism 32 onto the horizontal bed of the assembly machine (not shown). The backsheet 30 may, if desired, be an impervious plastic film.

The arrow 33 indicates the direction of flow of material during the assembly process, generally described as being the "machine direction" of the flow process. The arrow 34 indicates the "cross-machine" direction.

The apparatus may include an automatic splice unwind device (not shown) so that at high speeds the transfer of the material 30 from one roll 31 to an auxiliary roll (not shown) may take place automatically and without stopping the machine.

A preferred form of the backsheet material 30 is 32 cm (13") wide polyester spun-lace (i.e., hydro-entangled) material which may be printed or have primary (i.e. bright) colors.

Shortly after the web 30 begins its horizontal movement, a pair of adhesive "guns" apply strips of hot melt adhesive 36 to the web 30 as by conventional spray-applicating systems.

While the adhesive 36 is still tacky, waist elastic members, unwound from supply rolls 37 are deposited in intermittent strips 38 and 39 onto the tacky adhesive 36.

A preferred material for waist elastic members is a heat-shrinkable elastic material.

Because one of the elastic members as, for instance, 38, will be at the front waist portion of the garment, and 39 will be at the back waist portion of the garment, these two members 38 and 39 may be different color elastic to help the user of the garment identify the front and back portions. This may be particularly effective and critical for a child two to three years of age, for whom color identification is meaningful, but for whom word identification is not. For instance, the back elastic 39 may be colored blue and the front elastic 38 may be clear.

As the web 30 moves along the processing machine, it flows beneath another adhesive applicator 40, which may be a conventional slot-coating device, and which applies a barrier material 41.

A preferred adhesive material 41 which is applied by the applicator 40 is a clear hot-melt material.

This barrier material is applied intermittently in a rectangular pattern in a strip 9 cm (3-1/2 inches) wide and 45 cm (17-1/2 inches) long and approximately 25 to 50 microns (1 to 2 mils) thick. The long dimension of the barrier material is in the cross-machine direction, and the distance between the trailing edge of one application area and the leading edge of the followng application area is preferably 25 cm (10 inches).

It is to be understood that the dimensions given for this area may vary, as, for instance, the length may be less than 45 cm (17-1/2 inches) long but, in any event, the dimensions should be matched to the dimension of the absorbent core hereinafter to be described.

The area of the hot melt barrier material is indicated at 42 in the drawing.

Further downstream from the applicator 40, a roll of thermally-bonded absorbent core material 43 is unwound and cut into discreet portions which are applied directly as individual pieces 44 on top of the barrier material 42.

This thermally-bonded core material rpeferably is a combination of superabsorbent material, cellulose fluff, and thermally-bondable fibers, and the pieces 44 are applied to the hot-melt barrier material 42 while the barrier material 42 is still "tacky", and thus the barrier material not only serves to provide an impervious section on the web 30 directly beneath the core 44, but also holds the core 44 in place on the web 30.

When the selected web material 30 is pervious (such as spun-lace or cotton material), it is important to provide an impervious area beneath the absorbent core, and this is accomplished by applying the hot melt barrier material directly between the web 30 and the absorbent core 44.

At this point, downstream from the just-applied core 44, the web of coverstock material 45 is brought onto the machine.

This web 45 started in a roll 46 "off-machine" (i.e., not in-line with the process and equipment which has just been described). The web 45 is of pervious material and is unwound from the roll 46, and is preferably a lightweight nonwoven material having good formation and tactile properties. This means that preferably it has a "soft hand". Several types of this nonwoven material are available, and it may be thermally-bonded polyester or polypropylene fibers, spun bonded polypropylene, or the like.

After the web 45 is unwound from the roll 46, a plurality of hot-melt spray applicators (not shown) apply discrete sections of hot-melt adhesive from conventional spray systems to provide the adhesive areas 47-50. Two of these areas 47 and 48 are designed to hold the leg elastics, while the other two shorter areas 49 and 50 will hold the frontal elastics.

Downstream in the movement of the web 45, after the application of the adhesive portions 47-50, a plurality of leg and frontal elastics 51-54 of the heat shrinkable material previously described, are unwound from dispensers 55 and laid upon the adhesive portions 47-50. Two of these elastics 51 and 52 provide the leg elasticating mechanism, while the other two, 53 and 54, provide the frontal elastication mechanism.

It is preferred that these last-mentioned elastic members 51-54 also be of the clear, heat-shrinkable material provided by Minnesota Mining & Manufacturing Company. They are applied with a cut-and-space module (not shown).

The two strands which form the leg elastics 51 and 52 are preferably 33 cm (13 inches) long and 1.3 cm (1/2 inches) wide, while the two strands which form the frontal elastic are preferably 15 cm (6 inches) long and 1.3 cm (1/2 inches) wide.

Thus the two longer strands 51 and 52 extend through the crotch portion 13 to both the frontal portion 11 and the back portion 12 to provide the leg elastics 23 and 24 shown in Figure 1.

After the application of the elastic members to the web 45, all of which has been taking place on the top of the web, the web approaches the machine and is turned around a turning bar 56 so as to overlie the backsheet 30 with the elastic members underneath the coverstock 45 and, therefore, between the coverstock and the backsheet.

Just prior to the assembly of the coverstock on the backsheet, as at the area 57, an adhesive applicator 58 applies conventional hot melt through a conventional spray system in an appropriate pattern so as to bond the webs 30 and 45 together with the aforementioned elastics and absorbent core disposed therebetween.

The combining of the two webs in the area 57 may be done by a pair of rollers 59, or, if it is preferred not to use a hot melt for this assembly process, the item 59 may be an ultrasonic bonding section or a mechanical crimping section.

Because the hot melt system is well-known and has been well-tested, such application is preferred, although the other technologies are to be considered as suitble alternatives.

After the two webs have been assembled, a folding pan 60 causes the edge 61 of the assembled web to fold over and on top of and aligned with the edge 62 to provide a U-shaped product with the fold line 63 hereinafter defining the bottom of the crotch of the training pant.

This bi-sectional fold 64 is followed by a cutting section (not shown) which removes a portion 65 at spaced intervals along the bi-folded edge 63. This cutting may be done by a rotary die or rotary knife and, as will be seen, provides a cut-out portion in two adjacent garments being formed in the bi-sectional fold. In the preferred embodiment of the design, the cut-out portion has a cross-machine direction of 10 cm (4 inches).

Thereafter, a cutting and sealing mechanism (not shown) severs the bi-sectionally folded garment along the lines 66, while at the same time crmping or sealing the multi-layer folds in the areas 67 and 68 directly adjacent on each side of the cut-line 66. This cutting action provides the side flanges 14 and 15 which completes the waist portions 18 and 19 shown in Figure 1.

After the individual garments have been divided by the severing action just described, the garments pass through a heat tunnel, which causes the heat-shrinkable elastics heretofore described to become activated, and to complete the manufacturing process. The preferred length of time as the garments pass through the heat tunnel is approximately 10 seconds at a temperature of 93°C (200° F), but obviously the speed and temperature can change depending upon the preferred flow characteristics of the products through the assembly line.

After the garments have been completed, they can be packaged in substantially the same type of plastic bag packaging material well-known for the marketing of baby diapers. It is preferred that the packaging be of the compressed package type, so as to provide economies of space and materials.

It is clear, therefore, that we have provided a training garment which has many characteristics of previously known garments, but it should also be evident that the high-speed process of the present invention produces a unique garment because of the frontal elastics 25 and 26 which provide a better fit and a more cosmetic appearance to the finished garment.

## Claims

1. A process for providing a disposable training pant (10) for adolescent children and incontinent adults comprising the steps of
- providing a web (18,30) of backing material
- moving said web (18,30) along an assembly machine, and
- applying discrete sections (36) of adhesive on said web (18,30),
characterized by
- providing a web (43) of absorbent core material,
- separating said core web (43) into sections (44),
- depositing a waist elastic member (38, 39) on each of said sections (36) of adhesive,
- applying a discrete area (42) of barrier material (41) to said web (18,30) of backing material,
- depositing a section (44) of said core web (43) upon the aforementioned barrier material (41),
- providing a second web (45) of pervious coverstock material,
- applying discrete sections (47 to 50) of adhesive material transversely on said second web (45) to define at least a pair of leg elastic securement areas (47, 48) and a pair of frontal elastic securement areas (49, 50)
- providing a plurality of leg elastic members (51, 52) and a plurality of frontal elastic members (53, 54),
- depositing said leg elastic members (51, 52) and said frontal elastic members (53, 54) upon the adhesive in the respective securement areas (47 to 50),
- inverting said second web (45) and moving it onto the web (18, 30) of pervious backing material, in alignment therewith, and in synchronism therewith, so that the aforementioned leg and frontal elastic members (51 to 54) are disposed one on each side of the section (44) of core material (43),
- bonding said webs (30, 45) together,
- folding said bonded webs (30, 45, 64) longitudinally in half and aligning the opposed edges (61, 62) thereof,
- removing a section (65) of the bonded webs (20, 45, 64) along the fold line (63), and
- cutting said webs (30, 45, 64) along a line (66) transversely of said webs (30, 45, 64) at the middle of said removed section (65) and sealing the webs (30, 45, 64) on each side (67, 68) of said cutting line (66) to provide individual training pants (10).

2. The process according to Claim 1 wherein said elastic members (38, 39, 51 to 54) are made of heat activatible plastic material which is flat and non-elastic when applied to the respective webs (30, 45).

3. The process according to Claim 2 including passing said individual garments (10) through a heating chamber so as to activate the heat-activatable elastic.

4. The process according to Claim 3 wherein the heating chamber is at a temperature of between 80 and 100°C (180° and 210° F) and the residence time of the pants in the chamber is between 8 to 12 seconds.

5. The process of Claim 4 wherein the temperature of the heating chamber is 93° C (200° F) and the residence time of the pants in the chamber is 10 seconds.

6. The process according to one of the Claims 1 to 5 wherein the adhesive material (36, 47 to 50) is a hot-melt adhesive.

7. The process according to one of the Claims 1 to 6 wherein the bonding of the two webs (30, 45) is accomplished by using a hot-melt material, by mechanically crimping two webs together, by an ultrasonic means, or by sewing.

8. The process according to one of the Claims 1 to 7 wherein the cutting and sealing of the folded webs (30, 45) is accomplished simultaneously.

9. A training pant (10) for adolescent children and incontinent adults produced by the process according to one of the preceding claims, said pant having a front waist portion (11) with a frontal end, a rear waist portion (12) with a rear end, a crotch portion (13) side portions and leg openings in said side portions adjacent said crotch portion (13),
- a backsheet (18, 30)
- a porous topsheet (19, 45)
- an absorbent core (20, 43, 44) having a major and a minor dimension and disposed between the topsheet (19, 45) and the bakcsheet (18, 30) and between the leg openings,
- at least one leg elastic member (23, 24, 51, 52) between each major dimension of the absorbent core (20, 43, 44) and its adjacent leg opening,
- one end of each of said leg elastic members (23, 24, 51, 52) terminating in the front waist portion (11),
- a pair of frontal elastic tapes (25, 26, 53, 54) in the front waist portion, at least a portion of which tapes lie above the terminal ends of the leg elastic members (23, 24, 51, 52),
characterized by
- a barrier material (41, 42) disposed between the core (20, 43, 44) and the backsheet (18, 30).

10. A training pant according to Claim 9 wherein the frontal elastic tapes (25, 26, 53, 54) are separated laterally a distance greater than the distance between the leg elastic members (23, 24, 51, 52).

11. A training pant according to Claim 9 or 10 wherein the tension in the frontal elastic tapes (25, 26, 53, 54) is different from the tension in the leg elastic members (23, 24, 51, 52).

12. A training pant according to one of the Claims 9 to 11 including at least one waist elastic member (21, 39) in the front waist portion (11) between the frontal elastic tapes (25, 26, 53, 54) and the frontal end of the pant.

13. A training pant according to one of the Claims 9 to 12 including a waist elastic member (22, 38) in the rear waist portion (12) above the terminal ends of the leg elastic members (23, 24, 51, 52) and the rear end of the pant.

14. A training pant according to one of the Claims 9 to 13 wherein the absorbent core (20, 43, 44) is rectangular.

15. A training pant according to one of the Claims 9 to 14 wherein the backsheet (18, 30) is a porous material.

16. A training pant according to one of the Claims 9 to 15 wherein the porous backsheet (18, 30) and the porous topsheet (45) are both formed of hydro-entangled nonwoven webs.

17. A training pant according to one of the Claims 9 to 16 wherein the backsheet (18, 30) is a cotton material.

18. A training pant according to one of the Claims 9 to 17 wherein the leg elastic members (23, 24, 51, 52) are between 30 and 35 cm (12 and 14 inches) long.

19. A training pant according to Claim 18 wherein the leg elastic members (23, 24, 51, 52) are 25 cm (10 inches) long and the frontal elastic members (25, 26, 53, 54) are 15 cm (6 inches) long.

20. A training pant according to one of the Claims 9 to 19 wherein the frontal elastic members (25, 26, 53, 54) are between 10 and 18 cm (4 and 7 inches) long.

21. A training pant according to one of the Claims 9 to 20 wherein the absorbent core (20, 43, 44) is 9 cm (3.5 inches) wide and 45 cm (17.5 inches) long.

22. A training pant according to one of the Claims 9 to 21 wherein the absorbent core (20, 43, 44) is a combination of superabsorbent material, cellulose pulp and thermally-bondable fibers.

23. A training pant according to one of the Claims 9 to 22 wherein the elastic members (38, 39, 51 to 54) are a heat-shrinkable thermal-elastic material.

24. A training pant according to one of the Claims 9 to 23 wherein the backsheet (18, 30) and the topsheet (19, 45) are made of a polyester spunlace material.

25. A training pant according to one of the Claims 9 to 24 wherein the barrier material (41, 42) is a clear hot-melt adhesive material.

## Patentansprüche

1. Verfahren zur Herstellung einer wegwerfbaren Erziehungshose (10) für Jugendliche und an Inkontinenz leidende Erwachsene, welches die Schritte aufweist,
- Bereitstellen einer Bahn (18, 30) aus einem Trägermaterial,
- Bewegen der Bahn (18, 3O) längs einer Fertigungsmaschine und
- Aufbringen gesonderter Klebstoffabschnitte (36) auf die Bahn (18, 30),
gekennzeichnet durch
- Bereitstellen einer Bahn (43) aus absorbierendem Kernmaterial,
- Trennen der Kernbahn (43) in Abschnitte (44),
- Ablegen eines elastischen Hüftelements (38, 39) auf jedem der Klebstoffabschnitte (36),
- Aufbringen einer einzelnen Fläche (42) eines Sperrmaterials (41) auf die Bahn (18, 30) aus Trägermaterial,
- Ablegen eines Abschnitts (44) der Kernbahn (43) auf dem vorerwähnten Sperrmaterial (41),
- Bereitstellen einer zweiten Bahn (45) aus einem durchlässigen Abdeckpapiermaterial,
- Aufbringen getrennter Abschnitte (47 bis 50) aus Klebstoffmaterial quer auf die zweite Bahn (45) zur Bildung wenigstens eines Paares von elastischen Beinbefestigungsbereichen (47, 48) und eines Paares von elastischen frontalen Befestigungsbereichen (49, 50),
- Bereitstellen einer Vielzahl von elastischen Beinelementen (51, 52) und einer Vielzahl von elastischen frontalen Elementen (53, 54),
- Ablegen der elastischen Beinelemente (51, 52) und der elastischen frontalen Elemente (53, 54) auf dem Klebstoff in den jeweiligen Befestigungsbereichen (47 bis 50),
- Umkehren der zweiten Bahn (45) und Bewegen der Bahn auf die Bahn (18, 30) aus durchlässigem Trägermaterial in fluchtender Ausrichtung und synchron dazu, so daß von den erwähnten elastischen Beinelementen und elastischen frontalen Elementen (51 bis 54) jeweils eines auf jeder Seite des Abschnitts (44) des Kernmaterials (43) angeordnet ist,
- Verbinden der Bahnen (30, 45) miteinander,
- Falten der verbundenen Bahnen (30, 45, 64) in Längsrichtung auf die Hälfte und fluchtendes Ausrichten ihrer gegenüberliegenden Ränder (61, 62),
- Entfernen eines Abschnitts (65) der verbundenen Bahnen (20, 45, 64) längs der Faltlinie (63) und
- Schneiden der Bahnen (30, 45, 64) längs einer Linie (66) quer zu den Bahnen (30, 45, 64) an der Mitte des entfernten Abschnitts (65) und dichtes Verbinden der Bahnen (30, 45, 64) auf jeder Seite (67, 68) der Schnittlinie (66) zur Schaffung einzelner Erziehungshosen (10).

2. Verfahren nach Anspruch 1, bei welchem die elastischen Elemente (38, 39, 51 bis 54) aus wärmeaktivierbarem Kunststoffmaterial hergestellt sind, das eben und nicht elastisch ist, wenn es auf die jeweiligen Bahnen (30, 45) aufgebracht wird.

3. Verfahren nach Anspruch 2 einschließlich dem Durchführen einzelner Kleidungsstücke (10) durch eine Heizkammer, um die wärmeaktiverbaren elastischen Elemente zu aktivieren.

4. Verfahren nach Anspruch 3, bei welchem die Heizkammer eine Temperatur zwischen 80 und 100° C (180° und 210°F) hat und die Verweilzeit der Hosen in der Kammer zwischen 8 und 12 s liegt.

5. Verfahren nach Anspruch 4, bei welchem die Temperatur der Heizkammer 93° C (200° F) und die Verweilzeit der Hosen in der Kammer 10 s betragen.

6. Verfahren nach einem der Ansprüche 1 bis 5, bei welchem das Klebstoffmaterial (36, 47 bis 50) ein Heißschmelzklebstoff ist.

7. Verfahren nach einem der Ansprüche 1 bis 6, bei welchem das Verbinden der beiden Bahnen (30, 45) durch Verwenden eines Heißschmelzmaterials, durch mechanisches Zusammenfalten von zwei Bahnen, durch eine Ultraschalleinrichtung oder durch Nähen erreicht wird.

8. Verfahren nach einem der Ansprüche 1 bis 7, bei welchem das Schneiden und Abdichten der gefalteten Bahnen (30, 45) gleichzeitig erreicht wird.

9. Erziehungshose (10) für Jugendliche und an Inkontinenz leidende Erwachsene hergestellt nach dem Verfahren nach einem der vorhergehenden Ansprüche, wobei die Hose einen vorderen Hüftabschnitt (11) mit einem frontalen Ende, einen hinteren Hüftabschnitt (12) mit einem hinteren Ende, einen Zwickelabschnitt (13), Seitenabschnitte und Beinöffnungen in den Seitenabschnitten angrenzend an den Zwickelabschnitt (13),
- eine Unterbahn (18, 30),
- eine poröse Oberbahn (19, 45),
- einen absorbierenden Kern (20, 43, 44), der eine größere und eine kleinere Abmessung hat und zwischen der Oberbahn (19, 45) und der Unterbahn (18, 30) und zwischen den Beinöffnungen angeordnet ist,
- wenigstens ein elastisches Beinelement (23, 24, 51, 52) zwischen jeder größeren Abmessung des absorbierenden Kerns (20, 43, 44) und seiner angrenzenden Beinöffnung,
- wobei ein Ende eines jeden der elastischen Beinelemente (23, 24, 51, 52) in dem vorderen Hüftabschnitt (11) endet, und
- ein Paar von elastischen frontalen Bändern (25, 26, 53, 54) in dem vorderen Hüftabschnitt aufweist, wobei wenigstens ein Abschnitt der Bänder über den Abschlußenden der elastischen Beinelemente (23, 24, 51, 52) liegt,
gekennzeichnet durch
- ein Sperrmaterial (41, 42), das zwischen dem Kern (20, 43, 44) und der Unterbahn (18, 30) angeordnet ist.

10. Erziehungshose nach Anspruch 9, bei welcher die elastischen frontalen Bänder (25, 26, 53, 54) seitlich um einen Abstand getrennt sind, der größer ist als der Abstand zwischen den elastischen Beinelementen (23, 24, 51, 52).

11. Erziehungshose nach Anspruch 9 oder 10, bei welcher die Spannung in den elastischen frontalen Bändern (25, 26, 53, 54) verschieden ist von der Spannung in den elastischen Beinelementen (23, 24, 51, 52).

12. Erziehungshose nach einem der Ansprüche 9 bis 11 mit wenigstens einem elastischen Hüftelement (21, 39) in dem vorderen Hüftabschnitt (11) zwischen den elastischen frontalen Bändern (25, 26, 53, 54) und dem frontalen Ende der Hose.

13. Erziehungshose nach einem der Ansprüche 9 bis 12 mit einem elastischen Hüftelement (22, 38) in dem hinteren Hüftabschnitt (12) über den Abschlußenden der elastischen Beinelemente (23, 24, 51, 52) und dem hinteren Ende der Hose.

14. Erziehungshose nach einem der Ansprüche 9 bis 13, bei welcher der absorbierende Kern (20, 43, 44) rechteckig ist.

15. Erziehungshose nach einem der Ansprüche 9 bis 14, bei welcher die Unterbahn (18, 30) aus einem porösen Material besteht.

16. Erziehungshose nach einem der Ansprüche 9 bis 15, bei welcher die poröse Unterbahn (18, 30) und die poröse Oberbahn (45) jeweils aus durch Hydroverschlingung hergestellten Vliesbahnen bestehen.

17. Erziehungshose nach einem der Ansprüche 9 bis 16, bei welcher die Unterbahn (18, 30) aus einem Baumwollematerial besteht.

18. Erziehungshose nach einem der Ansprüche 9 bis 17, bei welcher die elastischen Beinelemente (23, 24, 51, 52) zwischen 30 und 35 cm (12 und 14 Zoll) lang sind.

19. Erziehungshose nach Anspruch 18, bei welcher die elastischen Beinelemente (23, 24, 51, 52) 25 cm (10 Zoll) lang und die elastischen frontalen Elemente (25, 26, 53, 54) 15 cm (6 Zoll) lang sind.

20. Erziehungshose nach einem der Ansprüche 9 bis 19, bei welcher die elastischen frontalen Elemente (25, 26, 53, 54) zwischen 10 und 18 cm (4 und 7 Zoll) lang sind.

21. Erziehungshose nach einem der Ansprüche 9 bis 20, bei welcher der absorbierende Kern (20, 43, 44) 9 cm (3,5 Zoll) breit und 45 cm (17,5 Zoll) lang ist.

22. Erziehungshose nach einem der Ansprüche 9 bis 21, bei welcher der absorbierende Kern (20, 43, 44) eine Kombination aus superabsorbierendem Material, Zellulosepulpe und thermisch bindbaren Fasern ist.

23. Erziehungshose nach einem der Ansprüche 9 bis 22, bei welcher die elastischen Elemente (38, 39, 51 bis 54) aus einem wärmeschrumpfbaren thermoelastischen Material bestehen.

24. Erziehungshose nach einem der Ansprüche 9 bis 23, bei welcher die Unterbahn (18, 30) und die Oberbahn (19, 45) aus einem Spunlace-Polyestermaterial bestehen.

25. Erziehungshose nach einem der Ansprüche 9 bis 24, bei welcher das Sperrmaterial (41, 42) ein durchsichtiges Heißschmelzklebstoffmaterial ist.

## Revendications

1. Procédé de fabrication d'une culotte d'apprentissage (10) jetable pour enfants adolescent, et adultes incontinents, ce procédé consistant à
- prendre un tissu (18, 30) fait d'une matière de support,
- déplacer ce tissu (18, 30) le long d'une machine d'assemblage et
- appliquer un nombre défni de filets de colle (36) sur ce tissu (18, 30),
ce procédé étant caractérisé en ce que
- on fournit un tissu (43) fait d'une matière constituant le noyau absorbant,
- on divise ce tissu constituant le noyau (43) en morceaux (44),
- on applique un élément élastique (38, 39) pour la taille sur chacun des filets de colle (36),
- on applique une surface définie (42) de matière de barrage (41) sur le tissu (18, 30) fait de matière de support,
- on applique un morceau (44) du tissu constituant le noyau (43) sur la matière de barrage (41) mentionnée ci-dessus,
- on fournit un deuxième tissu (45) fait d'une matière de couverture perméable,
- on applique des filets de colle (47 à 50), transversalement, sur le deuxième tissu (45) afin de définir au moins deux zones (47, 48) de fixation des élastiques pour les jambes et deux zones (49, 50) de fixation des élastiques du devant,
- on fournit plusieurs éléments élastiques (51, 52) pour les jambes et plusieurs éléments élastiques (53, 54) pour le devant,
- on dépose les éléments élastiques (51, 52) pour les jambes et les éléments élastiques (53, 54) pour le devant sur la colle dans chacune des zones de fixation (47 à 50) correspondantes,
- on retourne le deuxième tissu (45) et on l'amène sur le tissu (18, 30) fait de la matière de support perméable, tout en l'alignant et en le synchronisant avec celui-ci, de manière à ce que les éléments élastiques (51 à 54) pour les jambes et pour le devant, mentionnés ci-dessus, soient situés de part et d'autre du morceau (44) de matière (43) constituant le noyau,
- on colle ces deux tissus (30, 45) l'un sur l'autre,
- on plie en deux, dans le sens longitudinal, les deux tissus (30, 45, 64) collés tout en alignant leurs bords opposés (61, 62),
- on supprime une partie (65) des tissus collés (20, 45, 64) le long du pli 63) et
- on coupe les tissus (30, 45, 64) le long d'une ligne (66) transversale à ceux-ci (30, 45, 64) et passant au milieu de la partie supprimée (65) et on scelle les tissus (30, 45, 64) de chaque côté (67, 68) de la ligne de coupure (66) afin de former des culottes d'apprentissage (10) individuelles.

2. Procédé selon la revendication 1, dans lequel les éléments élastiques (38, 39, 51 à 54) sont faits d'une matière plastique à activation thermique qui est aplatie et non-élastique lorsqu'elle est déposée sur chacun des tissus (30, 45).

3. Procédé selon la revendication 2, comprenant le passage des vêtements individuels (10) dans une chambre chauffante de manière à activer l'élastique à activation thermique.

4. Procédé selon la revendication 3, dans lequel une température comprise entre 80 et 100 C (180 et 210 F) règne dans la chambre chauffante et dans lequel le temps de séjour des culottes dans la chambre est compris entre 8 et 12 secondes.

5. Procédé selon la revendication 4, dans lequel la température de la chambre chauffante est de 93 C (200 F) et le temps de séjour des culottes dans la chambre est de 10 secondes.

6. Procédé selon l'une des revendications 1 à 5, dans lequel l'adhésif (36, 47 à 50) est une colle thermofusible.

7. Procédé selon l'une des revendications 1 à 6, dans lequel la liaison entre les deux tissus (30, 45) est réalisée en utilisant une matière thermofusible, en gaufrant les deux tissus ensemble de manière mécanique, à l'aide de moyens ultrasoniques ou a les cousant.

8. Procédé selon l'une des revendications 1 à 7, dans lequel les opérations consistant à couper et à sceller les tissus pliés (30, 45) sont exécutées simultanément.

9. Culotte d'apprentissage (10) pour enfants adolescents et adultes incontinents, fabriquée selon l'une des revendications précédentes, culotte qui comprend une partie (11) couvrant le devant de la taille pourvue d'une extrémité antérieure, une partie (12) couvrant l'arrière de la taille pourvue d'une extrémité postérieure, une partie (13) correspondant à l'entrejambe, des parties latérales et des ouvertures pour les jambes formées dans les parties latérales adjacentes à la partie (13) correspondant à l'entrejambe,
- un voile de fond (18, 30),
- un voile supérieur (19, 45) poreux,
- un noyau absorbant (20, 43, 44) pourvu d'un grand et d'un petit côtés et situé entre le voile supérieur (19, 45) et le voile de fond (18, 30) et entre les ouvertures pour les jambes,
- au moins un élément élastique (23, 24, 51, 52) pour les jambes, situé entre chacun des grands côtés du noyau absorbant (20, 43, 44) et l'ouverture pour une jambe adjacente à celui-ci,
- chaque élément élastique (23, 24, 51, 52) pour les jambes ayant une extrémité qui se termine dans la partie (11) couvrant le devant de la taille,
- deux élastiques antérieurs (25, 26, 53, 54) situés dans la partie couvrant le devant de la taille, une partie au moins de ces élastiques étant située au-dessus des extrémités terminant les éléments élastiques (23, 24, 51, 52) pour les jambes,
cette culotte étant caractérisée par
- une matière de barrage (41, 42) située entre le noyau (20, 43, 44) et le voile de fond (18, 30).

10. Culotte d'apprentissage selon la revendication 9, dans laquelle les élastiques (25, 26, 53, 54) du devant sont séparés par une distance latérale qui est supérieure à la distance entre les éléments élastiques (23, 24, 51, 52) pour les jambes.

11. Culotte d'apprentissage selon l'une des revendications 9 ou 10, dans laquelle la tension des élastiques (25, 26, 53, 54) du devant est différente de la tension des éléments élastiques (23, 24, 51, 52) pour les jambes.

12. Culotte d'apprentissage selon l'une des revendications 9 à 11, comprenant, dans la partie couvrant le devant de la taille, au moins un élément élastique (21, 39) pour la taille situé dans la partie (11) couvrant le devant de la taille, entre les élastiques (25, 26, 53, 54) du devant et l'extrémité antérieure de la culotte.

13. Culotte d'apprentissage selon l'une des revendications 9 à 12, comprenant, dans la partie (12) couvrant l'arrière de la taille, un élément élastique (22, 38) pour la taille situé au-dessus des extrémités terminant les éléments élastiques (23, 24, 51, 52) pour les jambes, entre celles-ci et l'extrémité postérieure de la culotte.

14. Culotte d'apprentissage selon l'une des revendications 9 à 13, dans laquelle le noyau absorbant (20, 43, 44) est rectangulaire.

15. Culotte d'apprentissage selon l'une des revendications 9 à 14, dans laquelle le voile de fond (18, 30) est fait d'une matière poreuse.

16. Culotte d'apprentissage selon l'une des revendications 9 à 15, dans laquelle le voile de fond (18, 30) poreux et le voile supérieur (45) poreux sont faits tous les deux dans des tissus non-tissés, hydro-enchevêtrés ("hydro-entangled nonwoven webs").

17. Culotte d'apprentissage selon l'une des revendications 9 à 16, dans laquelle le voile de fond (18, 30) est fait d'une matière de coton.

18. Culotte d'apprentissage selon l'une des revendications 9 à 17, dans laquelle les éléments élastiques (23, 24, 51, 52) pour les jambes ont une longueur comprise entre 30 et 35 cm (entre 12 et 14 pouces).

19. Culotte d'apprentissage selon la revendication 18, dans laquelle les éléments élastiques (23, 24, 51, 52) pour les jambes ont une longueur de 25 cm (10 pouces) et les éléments élastiques (25, 26, 53, 54) du devant une longueur de 15 cm (6 pouces).

20. Culotte d'apprentissage selon l'une des revendications 9 à 19, dans laquelle les éléments élastiques (25, 26, 53, 54) du devant ont une longueur comprise entre 10 et 18 cm (entre 4 et 7 pouces).

21. Culotte d'apprentissage selon l'une des revendications 9 à 20, dans laquelle le noyau absorbant (20, 43, 44) mesure 9 cm (3,5 pouces) de large et 45 cm (17,5 pouces) de long.

22. Culotte d'apprentissage selon l'une des revendications 9 à 21, dans laquelle le noyau absorbant (20, 43, 44) est formé par une combinaison de matière superabsorbante, de pâte de cellulose et de fibres thermo-collables.

23. Culotte d'apprentissage selon l'une des revendications 9 à 22, dans laquelle les éléments élastiques (38, 39, 51 à 54) sont faits dans une matière thermo-élastique thermo-rétrécissable.

24. Culotte d'apprentissage selon l'une des revendications 9 à 23, dans laquelle le voile de fond (18, 30) et le voile supérieur (19, 45) sont faits d'une matière polyester du type "spunlaced".

25. Culotte d'apprentissage selon l'une des revendications 9 à 24, dans laquelle la matière de barrage (41, 42) est une matière adhésive thermofusible transparente.
